# EUROPEAN PATENT APPLICATION

(11) **EP 2 266 967 A1**
(43) Date of publication of application: **29.12.2010**
(21) Application number: 09450121.0
(22) Date of filing: 19.06.2009
(51) Int. Cl.: C07D 309/30, C07D 455/02

(54) **Intermediates for the preparation of octahydroquinolizines**

(71) Applicant: 55pharma Drug Discovery & Development AG, 1010 Wien (AT)
(72) Inventor: Adjoran, Immanuel, A-3430 Tulln (AT); Froble, Klaus, 42113 Wuppertal (AT)
(74) Representative: Schwarz, Albin

(57) **Abstract**

This invention relates to compounds used as intermediates for the synthesis of octahydroquinolizines in accordance with the following formula: 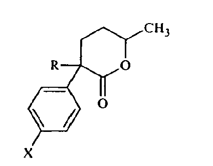 X = H, F; R= Methyl, Ethyl, nPropyl, nButyl

## Description

### FIELD OF THE INVENTION

This invention relates to compounds used as intermediates for the synthesis of octahydroquinolizines. Substituted octahydroquinolizines are for treatment or prevention of a number of diseases, including diabetes mellitus and its complications, for treatment or prevention of hyperlipidemia, for treatment or prevention of diabetic dyslipidemia, for treatment or prevention of the metabolic syndrome, for treatment or prevention of diseases related to metabolic dysfunction, for treatment or prevention of obesity or obesity-related diseases, for treatment or prevention of gastrointestinal dysfunction, for treatment or prevention of diseases mediated by opioid receptors located in the central nervous system or in the periphery, for treatment or prevention of opioid induced side effect e.g. constipation, nausea, vomiting and combinations of side effects of this type.

### BACKGROUND OF THE INVENTION

Differently substituted octahydroquinolizines are published as active pharmaceutical ingredients for use in indications listed above [W02007/050802 A *(Adolor Corp [US], Dolle Roland E [US], Le Bourdonnec Bertrand [US], 3 May 2007);* Kubo H. et al., Biol. Pharm.Bull. 23(9), 1114-1117 (2000*)].*

Therefore, it is of the utmost importance, to provide efficient synthetic access, which allows for stereoselective control in the course of the synthetic sequence leading to a variety of pharmacologically active octahydroquinolizines. It is common knowledge, although chemically similar in the backbone framework, specific changes in structure may result in changes in the pharmaceutical usefulness of structurally different octahydroquinolizine derivatives.

The compounds disclosed in this invention belong to a synthetic route using Alkyl-aryl-lactones of formula I as intermediates, which are used for, but not limited to, the synthesis of octahydroquinolizine derivatives. 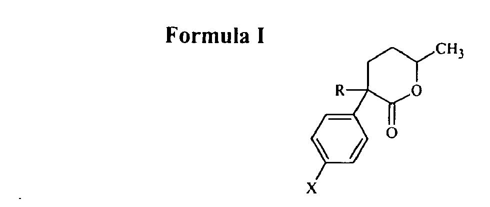 X = H, F; R= Methyl, Ethyl, nPropyl, nButyl

### SUMMARY OF THE INVENTION

The present invention is generally directed to a synthetic method using Alkyl-aryl-lactones intermediates, which are used for, but not limited to, the synthesis of substituted octahydroquinolizine derivatives, pharmaceutical compositions containing octahydroquinolizine derivatives and methods of their pharmaceutical use.

In one embodiment, the invention is directed to a method of preparation of the novel compound 5-hydroxy-2-phenylhexanenitrile **4,** its enantiomer, diastereomers or stereoisomeric mixtures following a known sequence of reactions, e.g. as described by Cossentini, M.; Seyden-Penne, J. Synth. Commun. 1985, 15(8), 689-696*,* including general methodology for keton reduction e.g. Organikum 2004 WILEY-VCH Verlag GmbH, Weinheim, p.: 568ff and general methodology for asymmetric ketone reduction e.g. Cordes, D. B. et. al. Eur. J. Org. Chem. 2005, 5289-529*,* B.Singaram et.al. Organic Process Research and Development 2006 (10), 949-958*,* K.Nakamura et.al.Tetrahedron:Asymmetry 14(2003), 2659-2681*.* The novel compound 5-hydroxy-2-phenylhexanenitrile **4,** its enantiomer, diastereomers or stereoisomeric mixtures can be transformed to the formerly described 6-methyl-3-phenyltetrahydro-2*H*-pyran-2-one **5** Kang, S. H.; Lee, J. H.; Lee, S. B. Tetrahedron Lett. 1998, 39, 59-62 by using general methods for lactonization e.g. Organikum 2004 WILEY-VCH Verlag GmbH, Weinheim, p.:475 and 500*.*

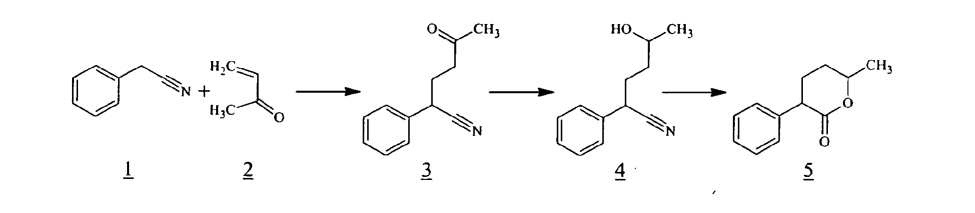

In another embodiment, the invention is directed to a method of preparation of the novel compounds 2-(4-fluorophenyl)-5-oxohexanenitrile **7**, 2-(4-fluorophenyl)-5-hydroxyhexanenitrile **8** and 3-(4-fluorophenyl)-6-methyltetrahydro-2*H*-pyran-2-one **9,** their respective enantiomers, diastereomers or stereoisomeric mixtures following a known sequence of reactions, e.g. as described by Cossentini, M.; Seyden-Penne, J Synth. Commun. 1985, 15(8), 689-696*,* including general methodology for keton reduction e.g. Organikum 2004 WILEY-VCH Verlag GmbH, Weinheim, p.: 568ff and general methodology for asymmetric ketone reduction e.g. Cordes, D. B. et. al. Eur. J. Org. Chem. 2005, 5289-529*,* B.Singaram et.al. Organic Process Research and Development 2006 (10), 949-958*,* K.Nakamura et.al.Tetrahedron:Asymmetry 14(2003), 2659-26815 and general methods for lactonization e.g. Organikum 2004 WILEY-VCH Verlag GmbH, Weinheim, p.:475 and 500*.*

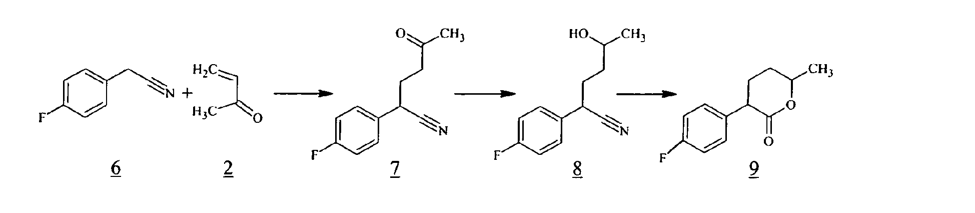

In yet another embodiment, the invention is directed to a method of preparation of the novel compounds 3,6-dimethyl-3-phenyltetrahydro-2*H*-pyran-2-one **10a** and 3-(4-fluorophenyl)-3,6-dimethyltetrahydro-2*H*-pyran-2-one **10b,** their respective enantiomers, diastereomers or stereoisomeric mixtures following a known sequence of reactions including the use of a strong base, but not limited to, e.g. lithiumdiisopropylamide and a alkylating reagent, but not limited to, like e.g. methyliodide as for example described in *Beck et al. Org. Synth. 1995, 72, 62.*

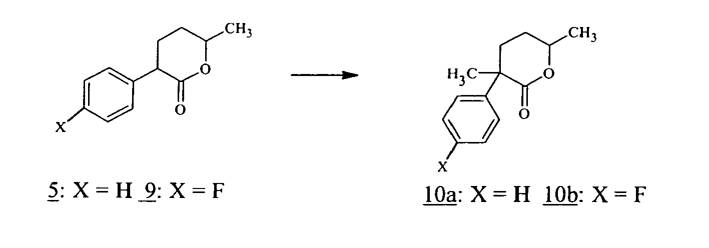

In a further embodiment, the invention is directed to a method of preparation of the novel compounds 5-hydroxy-2-methyl-2-phenylhexanamide **11a** and 2-(4-fluorophenyl)-5-hydroxy-2-methylhexanamide **11b,** 6-amino-5-methyl-5-phenylhexan-2-ol **12a** and 6-amino-5-(4-fluorophenyl)-5-methylhexan-2-ol **12b,** 3,6-dimethyl-3-phenyl-2,3,4,5-tetrahydropyridine **13a** and 3-(4-fluorophenyl)-3,6-dimethyl-2,3,4,5-tetrahydropyridine **13b,** their respective enantiomers, diastereomers or stereoisomeric mixtures following known synthetic methodology, e.g. as described in El-Farargy, A. F. Egypt. J. Pharm. Sci. 1991, 32(3-4), 565-74*;* Organikum 2004 WILEY-VCH Verlag GmbH, Weinheim, p.:570*;* Zibuck, R.; Streiber, J. Org. Synth. 1993, 71, 236*.* 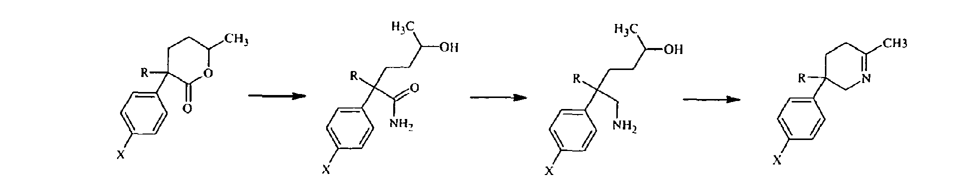 R= Methyl, Ethyl, nPropyl, nButyl

With R= Methyl:
10a: X = H 10b: X = F 11a: X = H 11b: X = F 12a: X = H 12b: X = F 13a: X = H 13b: X = F

The imines **13a,b** can be transformed to octahydroquinolizine derivatives according to Scheme A by methods described herein. 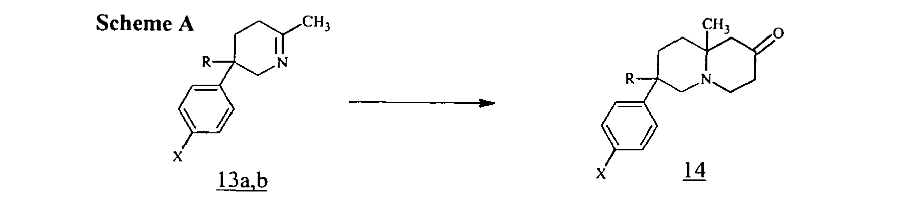 X = H, F; R= Methyl, Ethyl, nPropyl, nButyl
14a: X=H, R= Methyl; 14b: X=F, R= Methyl

These octahydroquinolizines can be used as active ingredients by themselves or they can be used as intermediates for the synthesis of further octahydroquinolizine derivatives for pharmaceutical use in the therapeutic fields as described above.

### METHODS OF PREPARATION AND EXAMPLES

If not stated otherwise, the following materials and solvents have been used: HPLC: Acetonitrile (ACN) LC-MS grade (Fluka); Water, LC-MS grade (Fluka); Formic acid, puriss. p.a. (eluent additive for LC-MS, Fluka); Dry solvents for chemical reactions: Tetrahydrofurane (THF), puriss., absolute, over molecular sieve H₂O ≤0.005%, ≥99.5% (GC) (Fluka)

If not stated otherwise, the following materials and solvents have been used for extraction and/or column chromatography: Ethyl acetate (EtOAc), GPR Rectapur (VWR Prolabo); Cyclohexane (CyclH), Toluene (Tol): Normapur (VWR Prolabo); Dichloromethane (CH₂Cl₂), Methanol (MeOH): GPR Rectapur (VWR Prolabo); Silica gel 60, 0.06-0.2 mm (Merck)

If not stated otherwise, the following reagents have been used for chemical reactions: 3-Buten-2-one, 99% (Aldrich); Iodomethane (MeI), purum: ≥99.0% (GC) (Fluka); Benzyl cyanide, 98% (Aldrich); Sodium sulfate (Na₂SO₄), purum p.a., anhydrous >99% (Fluka); Ammonium chloride (NH₄Cl), purum p.a., ≥99% (Fluka); Lithium diisopropylamide, 1,8 M solution in THF/Heptane/Ethylbenzene (Aldrich); Lithium aluminium hydride (LAH), reagent grade, 95%, powder (Aldrich); Sulfuric acid (H₂S0₄), 95-97% puriss. p.a. (Sigma-Aldrich); Sodium hydrogen carbonate (NaHCO₃) (Fluka); Magnesium sulfate anhydrous MgSO₄), puriss. p.a., drying agent, ≥98% (KT) (Fluka); Sodium carbonate (Na₂CO₃), purum, ≥98.0% (T) (Fluka); Acetic acid, purum 99% (Fluka); Sodium amide 95% (Aldrich); Hydrochloric acid (HCl), puriss. p.a., ACS reagent, fuming, ≥37% (Sigma-Aldrich); Chromium(VI) oxide, purum p.a., ≥99% (Fluka)

If not stated otherwise, the reaction products are identified and/or characterized by HPLC/MS. Instrumentation: SCL-10Avp, controller; DGU-20A5, degasser, FCV-10ALvp, low pressure gradient mixing unit, LC-10ADvp pump, SIL10ADvp, autosampler, SPD-M10Avp, PDA detector, LCMS 2010A MS detector (Shimadzu); SmartMix, gradient mixer with 350 µl mixing chamber (Knauer); N₂ LCMS 1, nitrogen generator (Claind); E2M28, two stage rotary vacuum pump (Edwards); Software: LabSolutions - LCMSolution Ver. 3.41 (Shimadzu); Sample preparation: Samples are weighted (1-3mg) and dissolved in acetonitrile/water (with 0.1% formic acid) = 1:9. Sample solutions are used undiluted for HPLC Method A, for HPLC Method B samples are diluted with acetonitrile/water (with 0.1 % formic acid) = 1:9 to a concentration of ∼0.05mg/ml. Solvents: solvent A: water with 0.1% formic acid, solvent B: acetonitrile with 0.1 % formic acid

Reaction products and stereoisomers are characterized by HPLC/MS via relative retention time in minutes after injection (RTT) applying the following methods. Detected ions are given in intensities in percent relative to base peak (100%). HPLC/MS Method A: Column: Synergi 4µ Polar-RP 80A 150x2.0 mm, with Security Guard Cartridge Polar-RP 4 x 2.0 mm (Phenomenex Inc.); flow: 0.5 ml/min; linear gradient (%A is the difference to 100%): start at 10% B, in 10 min to 100% B, then kept for 5 min at 100% B, then in 1 min to 10% B, then 7 min equilibration at 10% B; total run time: 23 min; PDA detector: wavelength: 190 - 600 nm, sampling rate: 1.56 Hz, MS detector: ionization mode: APCI positive, mass range: 150 - 600 ± 0.5 m/z; scan speed: 500 amu/sec; detector voltage: 1.25 kV; heat block temperature: 200 °C; CDL temperature: 250 °C; interface temperature:400°C; nebulizing gas flow: 2.5 L/min; dry gas pressure: 0.02 MPa. HPLC/MS Method B: Column: Synergi 4µ Polar-RP 80A 150x2.0 mm, with Security Guard Cartridge Polar-RP 4 x 2.0 mm (Phenomenex Inc.); flow: 0.5 ml/min; linear gradient (%A is the difference to 100%): start at 10% B, in 10 min to 100% B, then kept for 5 min at 100% B, then in 1 min to 10% B, then 7 min equilibration at 10% B; total run time: 23 min; PDA detector: wavelength: 190 - 600 nm, sampling rate: 1.56 Hz, MS detector: ionization mode: ESI positive, mass range: 150 - 600 ± 0.5 m/z; scan speed: 500 amu/sec; detector voltage: 1.25 kV; heat block temperature: 200 °C; CDL temperature: 250 °C; nebulizing gas flow: 1.5 L/min; dry gas pressure: 0.1 MPa. If not stated otherwise, RT stands for room temperature or ambient temperature, which typically lies between 20 and 25 °C.

### Preparation of 6-methyl-3-phenyltetrahydro-2H-pyran-2-one 5

6-methyl-3-phenyltetrahydro-2*H*-pyran-2-one **5** [Lit.: Kang, S. H.; Lee, J. H.; Lee, S. B. Tetrahedron Lett. 1998, 39, 59-62] is prepared by a sequence of standard reactions. Starting with benzyl cyanide and 3-buten-2-one 5-oxo-2-phenylhexanenitrile **3** is obtained by phase transfer catalysis [Lit.: Cossentini, M.; Seyden-Penne, J. Synth. Commun. 1985, 15(8), 689-696]. Keton reduction [Lit.: Organikum 2004 WILEY-VCH Verlag GmbH, Weinheim, p.: 568ff] of **3** delivers 5-hydroxy-2-phenylhexanenitrile **4**. Alkaline hydrolysis [Lit.: Organikum 2004 WILEY-VCH Verlag GmbH, Weinheim, p.:500*]* of **4** and lactonization [Lit.: Organikum 2004 WILEY-VCH Verlag GmbH, Weinheim, p.:475] of the obtained product delivers 6-methyl-3-phenyltetrahydro-2H-pyran-2-one **5.** HPLC/MS Method A: **4:** RTT = 8.5 [ms: 161, 190 (93%, M+H⁺), 231.2(21 %, M+ACN+H⁺)]; **5:** RTT = 9.0 [ms: 191.1 (30%, M+H⁺), 232.1 (M+ACN+H⁺), 381.2 (16%, 2*M+H⁺]

### Preparation of 3,6-dimethyl-3-phenyltetrahydro-2H-pyran-2-one 10a

6-methyl-3-phenyltetrahydro-2*H*-pyran-2-one **5** dissolved in dry THF is added dropwise to a 1.5eq. cooled LDA solution at -20°C in a dry and inert atmosphere (Argon). After stirring the reaction mixture for 1 hour at -20°C 1.5eq. iodomethane are added and the reaction mixture is warmed to RT. The reaction mixture is quenched with saturated ammonium chloride solution and extracted with ethyl acetate. The organic phase is dried over sodium sulfate, filtered and evaporated *in vac.* to dryness. Start-spot filtration (SiO₂; cyclohexane/ethyl actetate=9/1) of the obtained syrup delivers solid 3,6-dimethyl-3-phenyltetrahydro-2*H*-pyran-2-one **10a.** HPLC/MS Method A: **10a:** RTT = 9.6 [ms: 205.1 (17%, M+H⁺), 246.1 (M+ACN+H⁺), 409.2 (35%,2*M+H⁺]

### Preparation of 3,6-dimethyl-3-phenyl-2,3,4,5-tetrahydropyridine 13a

3,6-dimethyl-3-phenyltetrahydro-2*H*-pyran-2-one **10a** is dissolved in dry THF in a dry and inert atmosphere (Argon) and treated with 1.1 eq. sodium amide at room temperature. After stirring the reaction mixture for 16 hours it is quenched by the addition of water and extracted with CH₂Cl₂. The combined organic phases are washed with brine, dried over sodium sulfate, filtered and evaporated *in vac.* to dryness yielding 5-hydroxy-2-methyl-2-phenylhexanamide **11a.** Crude 5-hydroxy-2-methyl-2-phenylhexanamide **11a** is dissolved in dry THF in a dry and inert atmosphere (Argon) and treated with 6eq. lithium aluminum hydride. The reaction mixture is heated to reflux for 3 hours. The reaction is cautiously quenched with water and filtrated. The filtrate is extracted with CH₂Cl₂. The combined organic layers are washed with brine, dried over MgSO₄ and evaporated *in vac.* to dryness yielding 6-amino-5-methyl-5-phenylhexan-2-ol **12a.** 6-amino-5-methyl-5-phenylhexan-2-ol **12a** is dissolved in 2.9% sulphuric acid and 1.5eq. Jones reagents are added to the reaction mixture. After stirring for 16 hours at room temperature the reaction mixture is quenched with methanol and stirred for another hour. The reaction mixture is diluted with water and saturated aq. NaHCO₃ and filtered. The filtrate is extracted with ethyl acetate, the combined organic phases are washed with brine, dried over MgSO₄ and evaporated *in vac.* to dryness yielding 3,6-dimethyl-3-phenyl-2,3,4,5-tetrahydropyridine **13a.**
HPLC/MS Method A: **11a:** Isomere A: RTT = 6.8 [ms: 204.1 (M+H⁺), 222.1 (68%, M+H₃O⁺), 245.1 (20%, M+ACN+H⁺); Isomere B: RTT = 7.0 [ms: 204.1 (M+H⁺), 222.1 (37%, M+H₃O⁺), 245.1 (12%, M+ACN+H⁺); HPLC/MS Method B: **12a:** RTT = 4.1 [ms: 208 (M+H⁺)]; **13a:** RTT = 6.3 [ms: 188 (M+H⁺)]

### Preparation of 7,9a-dimethyl-7-phenyloctahydro-2H-quinolizin-2-one 14a and 7-(4-fluorophenyl)-7,9a-dimethyloctahydro-2H-quinolizin-2-one 14b

The crude 3,6-dimethyl-3-phenyl-2,3,4,5-tetrahydropyridine **13a** or 3-(4-fluorophenyl)-3,6-dimethyl-2,3,4,5-tetrahydropyridine **13b,** respectively, is dissolved in acetic acid and 2.3 eq. 3-buten-2-one is added. After stirring the reaction mixture at 50 °C for 24 hours it is diluted with toluene and the solvents are removed at 40°C under reduced pressure. The obtained syrup is distributed between saturated sodium carbonate solution and CH₂Cl₂, the organic phase is dried over sodium sulfate, filtered and evaporated *in vac.* to dryness. The crude product is purified by start-spot filtration (SiO₂; cyclohexane/ethyl actetate=9/1) and crystallized from cyclohexane yielding 7,9a-dimethyl-7-phenyloctahydro-2*H*-quinolizin-2-one **14a** or 7-(4-fluorophenyl)-7,9a-dimethyloctahydro-2*H*-quinolizin-2-one **14b,** respectively.
HPLC/MS Method A: **14a:** Isomere A: RTT = 6.7 [ms: 258.2 (M+H⁺); Isomere B: RTT = 6.9 [ms: 258.2 (M+H⁺)]; **14b:** Isomere A: RTT = 6.9 [ms: 276.2 (M+H⁺); Isomere B: RTT = 7.1
[ms: 276.2 (M+H⁺)]

## Claims

1. Alkyl-aryl-lactones according to formula I 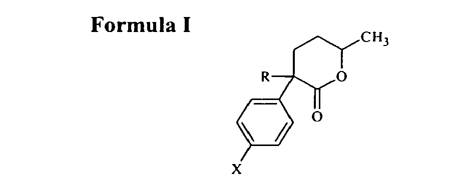 X = H, F; R= Methyl, Ethyl, nPropyl, nButyl
